# EUROPEAN PATENT APPLICATION

(11) **EP 2 338 879 A1**
(43) Date of publication of application: **29.06.2011**
(21) Application number: 09306230.5
(22) Date of filing: 15.12.2009
(51) Int. Cl.: C07D 209/08, C07D 213/81, C07D 471/04, A61K 31/404, A61K 31/437, A61K 31/4402, A61P 9/00

(54) **New phenylhydrazone derivatives and their use as pharmaceuticals**

(71) Applicant: Arteria, 30000 Nimes (FR)
(72) Inventor: Marguerie, M. Gérard, 94400, Vitry-sur-Seine (FR); Giethlen,, Bruno, 67120 Altorf (FR); Joubert, Muriel, 67400 Illkirch-Graffenstaden (FR); Garrido, Fabrice, 97210 Obernai (FR); Helin, Lionel, 30000 Nimes (FR)
(74) Representative: Maureau, Philippe

(57) **Abstract**

A compound of general formula (I) : 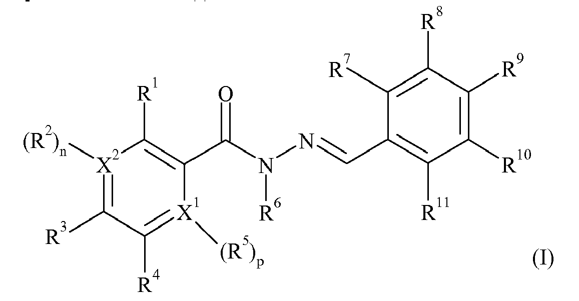 a process for preparing the same, a pharmaceutical composition comprising said compound comprising such compound and its use for treating or preventing diseases related to the metabolic syndrome such as hepatic and cardiovascular diseases.

## Description

The present invention relates to new phenylhydrazone derivatives, a process for preparing the same, pharmaceutical compositions comprising such compounds and their use for treating or preventing diseases related to the metabolic syndrome such as hepatic and cardiovascular diseases.

The metabolic syndrome, also designated as "X syndrome", insulin resistant syndrome or dysmetabolic syndrome, is a complex multi-genic pathology which can start to develop very early in life of the patient. This syndrome has become increasingly common in the industrialised countries It is characterized by a constellation of metabolic risk factors in one individual. The metabolic syndrome is closely associated with a generalized metabolic disorder called insulin resistance, in which tissue responsiveness to the normal action of insulin is impaired. It is a slow process generally developing over decades. This pathology implies various correlated or uncorrelated metabolic factors. It is generally considered as being a prediabetic stage of the disease.

The metabolic syndrome is characterised by metabolic disorders leading to abnormal levels of triglycerides, cholesterol, high density lipoprotein (HDL), glucose and insulin; but also to an excessive weight, a raised blood pressure, a pro-thrombotic state and/or a pro-inflammatory state. For example, the atherogenic dyslipidaemia of type II diabetes mellitus is characterised by an elevated fasting glucose (>100 mg/kg), a high level of triglycerides (greater than 150 mg/dl), a low high density lipoprotein cholesterol level (HDLc) less than 40 mg/dl for men and 50 mg/dl for women, and a variable low density lipoprotein cholesterol (LDLc) level (less than or greater than 100 mg/dl); whereas the hypertriglyceridaemia very often associated with obesity is characterised by a very high increase in the triglycerides (greater than 200 mg/dl) which enter into the structure of the lipoproteins.

The *Third report of the National Cholesterol Education Program Expert Panel on Detection: evaluation, and treatment of high blood cholesterol in adults (*Adult treatment panel III, 2002, Circulation, 106: 3143-3421, up-dated in Albertini K.G.M.M. et al 2009, Circulation, 120:1640-1645) has recently designated the metabolic syndrome as an independent factor raising the risks of cardiovascular diseases. All the above cited metabolic disorders indeed lead to development of pathologies such as cardiovascular and hepatic related diseases, among which angor, myocardial infarction, thrombosis, cardiac insufficiency, atherothrombosis, cerebral ischaemias, glaucoma and hepatic steatosis, insulin resistance, and impaired glucose tolerance. These pathologies can be associated with the generation of arterial plaques, hepatic vesicles, and hyperglycemia

The formation of an arterial plaque involves a gradual accumulation of oxidized lipoproteins accumulating in foamy macrophages and calcium on the arterial wall. The presence of numerous foamy macrophages in the plaque makes it vulnerable and causes episodes of rupture. Rupture of the atherosclerosis plaque and formation of a platelet thrombus are for their part acute processes responsible for the severe complications of the disease : coronary and cerebral infarction and sudden death. The severity of the disease therefore depends largely on the size of the plaque, its stability and the manner in which the thrombus is formed by rupture of this plaque. This phenomenon often involves a chronic inflammatory state in addition to an immune response.

In addition to their implication in atherothrombosis, factors of the metabolic syndrome are also associated with the development of diabetic related diseases. When a subject suffers from both, dyslipydemia and insulin resistance, the risks of having a cardiovascular collapse drastically increase. (Alexander CM, et col. - NCEP defined metabolic syndrome, diabetes and prevalence of coronary heart disease among NHANES IIIparticipants age 50 years and older, in Diabetes 2003, 52, 1210-1214).

It is generally accepted that both, the generation of arterial plaques, insulin resistance or impaired glucose tolerance can be directly correlated with the level of circulating oxidized lipoproteins which are true cardiovascular pathogen agents and represent a link between atherosclerosis and diabetes. Association between oxidized low density lipoproteins (LDL), obesity and type 2 diabetes has indeed been demonstrated and several lines of evidence have demonstrated that the level of oxidized LDL is significantly higher in patients with type diabetes (*See for instance,* Ehara S. Et al 2001, Circulation Res. 103-1955-1960*,* Schwenke D.C. et al 2003 Diabetes care 26:1149-1155*,* Njajou O. T. et al 2009 Diabetes Metab. Res. Rev.25:733-739*,* Sigurdardottir C. Et al 2002, J. Int. Med. 252: 440-447*).* These oxidized lipoproteins are captured by monocytes/marcophages, muscle cells, hepatocytes, and endothelial cells and their excessive accumulation leads to the appearance of numerous intracellular lipidic vesicles, lipotoxicity, peroxide accumulation and decreased gycolysis (Sukharov S. 2009 Circulation Research, 99:191-200)*.* Such lipotoxicity initiates cellular apoptosis and leads to the appearance of vulnerable plaques favouring ruptures and atherothrombosis as well as glucose metabolism dysfunctions. The cellular receptor of these oxidised lipoproteins belongs to a family of membrane receivers commonly called "scavengers family". For example, the SRAI, the SRBI, the LOXI and the CD36 are part of this family.

The physiopathology of these receivers and their control in developing of atherosclerosis has been studied. For example, it has been demonstrated in Febbraio et col. in J. Clin. Invest. 2000, 105, 1049-1057 that CD36 was a major receiver of oxidised lipoproteins at the level of macrophages and endothelial cells. Invalidating the coding gene of this receiver in atherogenosis mouse leads to an inhibition of more than 77% of the arterial plaques. It has also been demonstrated that CD36 is an example of scavenger establishing the link between hyperlipidemia, oxidative stress, insulin resistance and cardiac insufficiency. In addition, it is also commonly accepted that hyperlipidemia associated to hyperglycemia stimulate macrophage proliferation and the growth of arterial plaques.

Consequently, compounds allowing a control and an inhibition of the capture of the oxidised lipoproteins by macrophage muscle cells or endothelial cells might be very useful in the treatment and/or prevention of diabetic associated cardio-vascular diseases.

In spite of highly active pharmacological research and major advances in the fields of surgery, cardiovascular diseases, coronary accidents, cerebral ischaemias, diabetes and hepatic steatosis remain the main cause of deaths or invalidities in the industrialised countries.

To date, different therapeutic options are available for treating or preventing these diseases.

Hypolipaemic agents such as statins or ezetimibe possess recognised efficacy. Statins are inhibitors of 3-hydroxy-methylglutaryl coenzyme A reductase which is directly involved in cholesterol synthesis. Statins effectively reduce the cholesterol level and to a more limited degree, the triglyceride level. The problem with this approach is that atherogenic dyslipidemia of insulin resistant patients does not respond well to statin therapy.

Other agents such as ezetimibe, less efficient than statins, inhibit intestinal absorption of cholesterol. These molecules are therefore recommended as primary and secondary prevention for the majority of patients with a high LDLc level. The clinical trials have shown however that the medical benefit of hypolipidaemic agents, with regard to the cardiovascular risk, is only 30 to 35%. Their use is sometimes accompanied by undesirable adverse events which require treatment withdrawal. In many cases, muscular involvement, hepatic toxicity and intolerance phenomena are observed.

Fibrates or fibric acid derivatives, or nicotinic acid derivatives such as niacines, are also recommended for the treatment of atherogenic dyslipidaemias. Dyslipidaemias affect different patients with complex lipid profiles : a low cholesterol level, high triglyceride levels and low HDLc levels. The use of fibrates reduces the risk of cardiovascular accidents by approximately 40%. Their use is unfortunately accompanied in many patients by undesirable effects due to intolerance, hepatic toxicity and muscle involvement, these secondary effects being still increased in long-term if these compounds are associated with statines. Accordingly, such association is only used for treating high-risks patients.

The thrombotic accident resulting from rupture of an arterial plaque is generally treated with antithrombotic agents such as acetylsalicylic acid, thienopyridins or thyanopyridins.

New compounds are therefore needed which are capable of treating both lipid abnormalities leading to atherosclerosis and insulin resistance leading to hyperglycemia both inducing high risks of cardiovascular diseases.

Japanese patent application JP 11-110637 discloses a broad family of acetylhydrazone derivatives of general formula encompassing the compounds of the present invention, and their use for treating various diabetes complications and senile diseases. However, the compounds of the present invention are not specifically disclosed in that patent application.

International patent application WO 2005/082882 discloses a broad family of hydrazide derivatives of general formula encompassing the compounds of the present invention, and their use for treating cardiovascular diseases. However, the compounds of the present invention are not specifically disclosed in that patent application.

In addition, arylhydrazone derivatives disclosed in patent applications JP 11/110637 and WO 2005/082882 have shown to be genotoxic, which is a strong drawback since the therapeutical use of such compounds for treating or preventing cardiovascular diseases implies a chronical and long-term administration of the latter.

Accordingly, it is of high relevance to identify new compounds that are at least as efficient as said compounds and which do not evidence genotoxicity.

The present invention precisely aims at new phenylhydrazone derivatives useful as an active agent in pharmaceutical compositions, especially for treating or preventing of cardiovascular diseases, and which do not evidence any genotoxicity.

Accordingly, the present invention relates to a new phenylhydrazone derivative of general formula (I) : 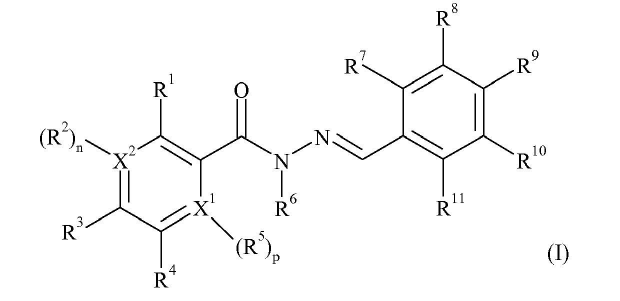 in which :
- X¹ and X² are chosen independently of each other as being a carbon atom or a nitrogen atom;
- n and p are chosen independently of each other as being 0 or 1;
- R¹, R⁴ and R⁵ are chosen independently of each other as being a hydrogen atom, a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
- R² and R³ are chosen independently of each other as being a hydrogen atom, a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
   or R² and R³ may together form a 3-, 4-, 5- or 6-membered carbocycle or heterocycle optionally substituted with one or further substituants chosen among a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
- R⁶ is chosen as being a hydrogen atom, a hydroxy group or a C₁-C₈-alkyl;
- R⁷, R⁸, R⁹, R¹⁰ and R¹¹ are chosen independently of each other as being a hydrogen atom, a halogen atom, a hydroxy group, a carboxyl group, -OR¹² or - COR¹², in which R¹² represents an amino group, a C₁-C₈-alkyl or -NR¹³R¹⁴ in which R¹³ and R¹⁴, are chosen independently of each other as being a halogen atom, a C₁-C₈-alkyl, an aryl radical or R¹³ and R¹⁴ may together form a 3-, 4-, 5- or 6-membered non-fused heterocycle optionally substituted with one or further substituants chosen among a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl; as well as its pharmaceutically acceptable salts.

In the context of the present invention :
- halogen means chlorine, bromine, iodine or fluorine; .
- carboxy means -C(=O)OH ;
- carbonyl means -C(=O)- ;
- an alkyl group, an alkenyl group, and an alkynyl group as well as moieties containing these terms, can be linear or branched;
- "carbocycle" designates any saturated or partially unsaturated or aromatic ring containing exclusively carbon atoms. Preferably, "carbocycle" designates a phenyl group;
- "heterocycle" designates any saturated or partially unsaturated or aromatic ring containing one or two heteroatoms chosen among N, O and S which can be identical or different. Preferably, "heterocycle" designates any saturated or partially unsaturated or aromatic ring containing one or two nitrogen atoms. More preferably "heterocycle" designates a pyridine moiety, a pyrimidine moiety, a pyrazine moiety, a pyridazine moiety, a pyrrole moiety, an imidazole moiety or a pyrazole moiety;
- "pharmaceutically acceptable salts" designate pharmaceutical type acids which are tolerated physiologically among which the salts of acetic, hydrochloric, cinnamic, citric, formic, hydrobromic, hydrolodic, hydrofluoric, malonic, methanesulphconic, oxalic, picric, maleic, lactic, nicotinic, phenylacetic, phosphoric, succinic and tartric acid, ammonium, diethylamine, piperazine, nicotinamide, urea, sodium, potassium, calcium, magnesium, zinc, lithium, methylamino, dimethylamino, trimethylamino and tris(hydroxymethyl)aminomethane salts; and
- a "pharmaceutically effective amount" means an amount of a compound or of a composition which is capable of alleviating the symptoms of the various pathological conditions herein described.

Compounds of the present invention inhibit the formation of foamy macrophage cells and reduce the apparition of metabolic disorders due to the metabolic syndrome. In addition, the compounds of the invention have not shown any mutagen activity when tested with an elementary AMES type test or with chromosomal aberration test. Compounds of the present invention possess the capacity of reducing both the formation of lipid loaded cells and hyperglycemia. They are therefore of great interest for treating atherogenic diabetes and its related cardiovascular and hepatic diseases.

The present invention relates to a compound of general formula (I). Preferably compound of general formula (I) according to the present invention have the following characteristics, taken individually or in combination :
- R¹ is chosen as being a hydrogen atom, a halogen atom, a nitro group, an amino group, a cyano group, a hydroxy group, a carboxyl group, a C₁-C₈-alkyl sucha as a methyl group or an ethyl group; a C₂-C₈-alkenyl; a C₃-C₈-alkynyl; a C₁-C₆-halogenoalkyl having 1 to 5 halogen atoms such as a trifluoromethyl group; more preferably R¹ is chosen as being a hydrogen atom;
- R² is chosen as being a hydrogen atom, a halogen atom, a nitro group, an amino group, a cyano group, a hydroxy group, a carboxyl group, a C₁-C₈-alkyl sucha as a methyl group or an ethyl group; a C₂-C₈-alkenyl; a C₃-C₈-alkynyl; a C₁-C₆-halogenoalkyl having 1 to 5 halogen atoms such as a trifluoromethyl group; more preferably R² is chosen as being a hydrogen atom;
- R³ is chosen as being a hydrogen atom, a halogen atom, a nitro group, an amino group, a cyano group, a hydroxy group, a carboxyl group, a C₁-C₈-alkyl sucha as a methyl group or an ethyl group; a C₂-C₈-alkenyl; a C₃-C₈-alkynyl; a C₁-C₆-halogenoalkyl having 1 to 5 halogen atoms such as a trifluoromethyl group; more preferably R³ is chosen as being a hydrogen atom or a trifluoromethyl group
- R² and R³ form together a phenyl, a pyridine moiety, a pyrimidine moiety, a pyrazine moiety, a pyridazine moiety, a pyrrole moiety, an imidazole moiety or a pyrazole moiety, each of which being optionally substituted with one or further substituants chosen among a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
- R⁴ is chosen as being a hydrogen atom, a halogen atom, a nitro group, an amino group, a cyano group, a hydroxy group, a carboxyl group, a C₁-C₈-alkyl sucha as a methyl group or an ethyl group; a C₂-C₈-alkenyl; a C₃-C₈-alkynyl; a C₁-C₆-halogenoalkyl having 1 to 5 halogen atoms such as a trifluoromethyl group; more preferably R⁴ is chosen as being a hydrogen atom;
- R⁵ is chosen as being a hydrogen atom, a halogen atom, a nitro group, an amino group, a cyano group, a hydroxy group, a carboxyl group, a C₁-C₈-alkyl such as a methyl group or an ethyl group; a C₂-C₈-alkenyl; a C₃-C₈-alkynyl; a C₁-C₆-halogenoalkyl having 1 to 5 halogen atoms such as a trifluoromethyl group; more preferably R⁵ is chosen as being a hydrogen atom;
- R⁶ is chosen as being a hydrogen atom;
- R⁷ is chosen as being a hydrogen atom, a halogen atom, a hydroxy group, a carboxyl group, -OR¹² or -COR¹², in which R¹² represents an amino group or a C₁-C₈-alkyl such as a methyl group or an ethyl group; more preferably R⁷ is chosen as being a hydrogen atom, a halogen atom, a hydroxy group, -OR1² in which R¹² is methyl or ethyl;
- R⁸ is chosen as being a hydrogen atom, a halogen atom, a hydroxy group, a carboxyl group, -OR¹² or -COR¹², in which R¹² represents an amino group or a C₁-C₈-alkyl such as a methyl group or an ethyl group; more preferably R⁸ is chosen as being a hydrogen atom, a halogen atom or a hydroxy group;
- R⁹ is chosen as being a hydrogen atom, a halogen atom, a hydroxy group, a carboxyl group, -OR¹² or -COR¹², in which R¹² represents an amino group or a C₁-C₈-alkyl such as a methyl group or an ethyl group; more preferably R⁹ is chosen as being a hydrogen atom, a halogen atom, a hydroxy group, -OR¹² in which R¹² is methyl or ethyl;
- R¹⁰is chosen as being is chosen as being a hydrogen atom, a halogen atom, a hydroxy group, a carboxyl group, -OR¹² or -COR¹², in which R¹² represents an amino group or a C₁-C₈-alkyl such as a methyl group or an ethyl group; more preferably R¹⁰ is chosen as being a hydrogen atom, a halogen atom or a hydroxy group;
   and/or
- R¹¹ is chosen as being a hydrogen atom, a halogen atom, a hydroxy group, a carboxyl group, -OR¹² or -COR¹², in which R¹² represents an amino group or a C₁-C₈-alkyl such as a methyl group or an ethyl group; more preferably R¹¹ is chosen as being a hydrogen atom, a halogen atom, a hydroxy group, -OR¹² in which R¹² is methyl or ethyl.

The present invention further relates to a compound of formula (I-a) : 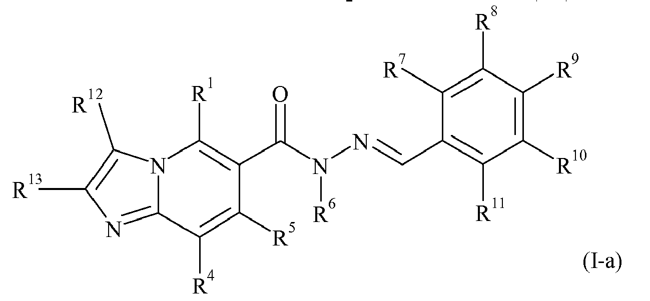 in which :
- R¹, R⁴, R⁵, R⁶, R⁷, R⁸, R⁹, R¹⁰ and R¹¹ are as defined above; and
- R¹² and R¹³ are chosen independently of each other as being a hydrogen atom, an amino group, a hydroxy group, a C₁-C₈-alkoxy, a group of formula (CH₂)ᵣ-(Y)ₛ-(CH₂)t-Z in which r and t are chosen independently of each other as being 0, 1, 2, 3, 4 or 5; s is 0 or 1; Y is chosen as being an oxygen atom, an amino group, a carboxy group and Z is chosen as being a C₁-C₈-alkyl such as methyl or ethyl group, or a C₁-C₆-halogenoalkyl having 1 to 5 halogen atoms such as trifluoromethyl group.

The present invention further relates to a compound of formula (I-b) : 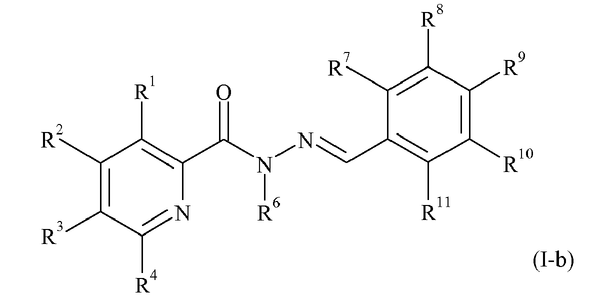 in which R¹ R², R³, R⁴, R⁶, R⁷, R⁸, R⁹ R¹⁰ and R¹¹ are as defined above.

The present invention further relates to a compound of formula (I-c) : 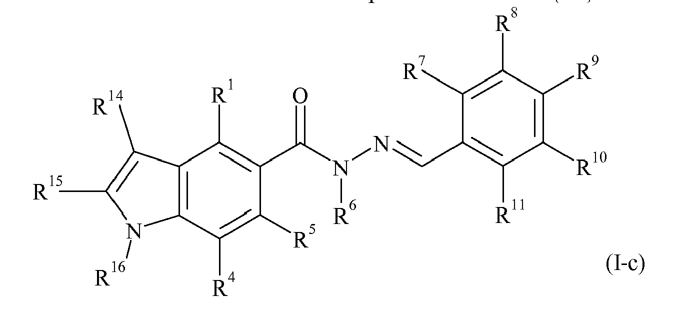 in which :
- R¹, R⁴, R⁵, R⁶, R⁷, R⁸, R⁹, R¹⁰ and R¹¹ are as defined above; and
- R¹⁴, R¹⁵ and R¹⁶ are chosen independently of each other as being a hydrogen atom, an amino group, a hydroxy group, a C₁-C₈-alkoxy, a group of formula (CH₂)ᵣ-(Y)ₛ-(CH₂)ₜ₋Z in which r and t are chosen independently of each other as being 0, 1, 2, 3, 4 or 5; s is 0 or 1; Y is chosen as being an oxygen atom, an amino group, a carboxy group and Z is chosen as being a C₁-C₈-alkyl such as methyl or ethyl group, or a C₁-C₆-halogenoalkyl having 1 to 5 halogen atoms such as trifluoromethyl group.

Compounds according to the present invention may be prepared according to reaction known to the skilled artisan. The present invention therefore also relates to a process for the preparation of the compound of general formula (I). Thus, according to a further aspect of the present invention there is provided a process for the preparation of compound of formula (I) as defined above according to the following reaction scheme : 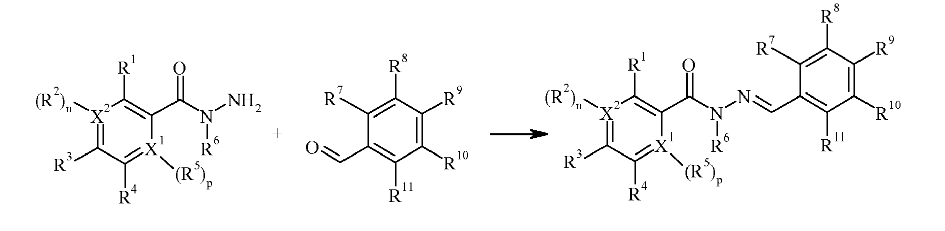 in which R¹ to R¹¹, X¹, X², n and p are as defined above;
wherein said process is conducted in an aprotic solvent, in the presence of a tertiary amine base and at a temperature of from 0°C to 50°C.

Preferably, the process according to the present in conducted under the following experimental conditions, taken individually or in combination :
- the aprotic solvent is chosen as being anhydric dimethylformamide (DMF) or ethanol;
- the tertiary amine base is chosen as being diisopropylethylamine (DIEA);
- the process is conducted at the room temperature; and/or
- the process is conducted under an inert atmosphere.

The present invention also relates to a pharmaceutical composition comprising, as an active ingredient, a pharmaceutically effective amount of a compound of general formula (I) as defined above. Preferably, the pharmaceutical composition according to the present invention contain from 0,5 to 20 mg of a compound of formula (I) as defined above.

The present invention also relates to a pharmaceutical composition comprising a pharmaceutically effective amount of a compound general formula (I) associated to one or further active ingredients chosen from hypolipaemic agents reducing cholesterol synthesis such as "statins"; angiotensin II converting enzyme inhibitors such as losartan; anticalcium agents; antithrombotics; beta blockers; inhibitors of the members of the class of peroxisome proliferator activated receptors (the PPAR class); inhibitors of triglyceride synthesis or metabolism such as the fenofibrates; agents capable of increasing insulin resistance such as the troglitazones or the pioglitazones.

The pharmaceutical compositions according to the present invention may be formulated under any form generally used in the pharmaceutical field. As an example, these may involve pharmaceutical vectors such as salts or electrolytes, salts of scorbic acid, water or buffered solutions, colloidal solutions, substances based on cellulose, polyethylene glycol, polyacrylates, waxes, proteins or any other substance capable of dissolving or rendering the active compound available for therapeutic action.
The compositions of the present invention may be administered in injectable form or via the oral or parenteral route, via the nasal route in spray form, via the rectal or vaginal route, by implantation of a reservoir or dispensers or in any other pharmaceutical form used in the pharmaceutical field. The injectable forms of these compositions may be aqueous or oily suspensions. These suspensions may be formulated according to any process used in this field by using non-toxic solvents or diluents such as 1,3-butanediol for example. Among the acceptable solvents, it is possible to use water, buffered solutions, Ringer solutions, or isotonic salt solutions. Other acceptable diluents may be formed of synthetic mono or di-glycerides, long-chain alcohols, or dispersants such as carboxymethyl cellulose or any other diluent or emulsifier used in formation of pharmaceutical suspensions.
The pharmaceutical compositions of the present invention administered via the oral route may be in the form of capsules, tablets or aqueous suspensions or in the form of emulsions. These formulations may possibly contain chemical compounds intended to attenuate or improve the taste.
The pharmaceutical compositions of the present invention may be administered in suppository form by mixing the product with a non-irritant, non-allergic, excipient, solid at ambient temperature and liquid at rectal temperature in order to release the active compound. Such formulations may for example use beeswax, polyethylene glycols or cocoa butter.

The pharmaceutical compositions according to the present invention are useful for inhibiting the formation of foamy macrophage cells and reducing the apparition of metabolic disorders due to the metabolic syndrome. They possess the property of reducing weight gain due to accumulation of abdominal fat, of reducing the increase in the total cholesterol and free cholesterol level and deposit of triglycerides on the arterial wall and of reducing accumulation of macrophages in the atheromatous plaques. Accordingly, the present invention also relates to a pharmaceutical composition as defined above for the treatment or the prevention of diseases associated with lipid metabolism disorders among which hypercholesterolaemia, hypertriglyceridaemia, dyslipoproteinaemia, chylomicronaemia, lipodystrophy and hyperglycaemia. The present invention also relates to a pharmaceutical composition as defined above for the treatment or the prevention of disorders associated with these dysfunctions, in particular obesity, type II diabetes mellitus, insulin resistance, impaired glucose tolerance and associated cardiovascular diseases among whichdiastolic and systolic dysfunction with or without ventricular dysfunction, atherosclerosis, heart failure, cerebral ischaemia (stroke) and hepatic steatosis.

Furthermore, since the pharmaceutical compositions according to the present invention also possess the property of reducing the lipid vesicle accumulation the present invention also relates to a pharmaceutical composition for the treatment or the prevention of liver steatosis and lipid vesicle accumulation.

Furthermore, since the pharmaceutical compositions according to the present invention also possess the property of reducing stenosis of the arterial wall, the present invention also relates to a pharmaceutical composition for the treatment or the prevention of restenosis.

The present invention further relates to the use of a compound of formula (I) as defined above for the preparation of a medicament for the preventive or curative treatment of diseases associated with lipid metabolism disorders among which hypercholesterolaemia, hypertriglyceridaemia, dyslipoproteinaemia, chylomicronaemia, lipodystrophy and hyperglycaemia. The present invention also relates to the use of a compound of formula (I) as defined above for the preparation of a medicament for the preventive or curative treatment of disorders associated with these dysfunctions, in particular obesity, type II diabetes mellitus, insulin resistance and impaired glucose tolerance and associated cardiovascular diseases among which diastolic and systolic dysfunction with or without ventricular dysfunction, atherosclerosis, heart failure, cerebral ischaemia (stroke) and hepatic steatosis.

Furthermore, since the pharmaceutical compositions according to the present invention also possess the property of reducing the lipid vesicle accumulation the present invention also relates to the use of a compound of formula (I) as defined above for the preparation of a medicament for the preventive or curative treatment of liver steatosis and lipid vesicle accumulation.

Furthermore, since the compound of formula (I) according to the present invention also possess the property of reducing stenosis of the arterial wall, the present invention also relates to the use of a compound of formula (I) as defined above for the preparation of a medicament for the preventive or curative treatment of restenosis.

Finally, the present invention further relates to a method of preventively or curatively treating diseases associated with lipid metabolism disorders among which hypercholesterolaemia, hypertriglyceridaemia, dyslipoproteinaemia, chylomicronaemia, lipodystrophy and hyperglycaemia. The present invention also relates to a method for preventively or curatively treating of disorders associated with these dysfunctions, in particular obesity, type II diabetes mellitus, insulin resistance impaired glucose tolerance and associated cardiovascular diseases among which diastolic and systolic dysfunction with or without ventricular dysfunction, atherosclerosis, heart failure, cerebral ischaemia (stroke) and hepatic steatosis; administering to an individual a compound of formula (I) as defined above.

Furthermore, since the pharmaceutical compositions according to the present invention also possess the property of reducing the lipid vesicle accumulation the present invention also relates to a method for preventively or curatively treating liver steatosis and lipid vesicle administering to an individual a compound of formula (I) as defined above.

Furthermore, since the compound of formula (I) according to the present invention also possess the property of reducing stenosis of the arterial wall, the present invention also relates to a method for preventively or curatively treating restenosis administering to an individual a compound of formula (I) as defined above.

The present invention will now be illustrated in a non-limited manner with reference to the following tables of compounds and examples.

### Example 1 : Process for the preparation of compounds according to the invention

### Example 1.1 - Preparation of [1-(2-hydroxy-4,6-diméthoxy-phenyl)-methylidene]-imidazo[1,2-alpyridine-6-carbohvdrazide (Compound 1)

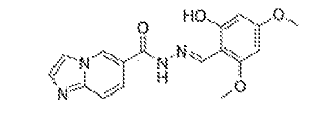

Into a dry tricol flask equipped with magnetic stirring, 176 mg of commercial acid imidazol[1,2]pyridine-6-carboxylic hydrazide (1.0 mmol) dissolved in 5 ml of anhydrous DMF is introduced.
After addition of 110 µl (1.5 mmol) of diisopropylethylamine (DIEA), the solution is stirred for 10 minutes at ambient temperature.
To this solution, 182 mg (1.0 mmol) 4,6-dimethoxylsalicyladehyde is added and the medium is stirred at ambient temperature for 24 hours. The progress of the reaction is monitored by HPLC analysis until complete consumption of the starting material. After evaporation of the solvent, the solid residue obtained is crystallized in acetonitrile dissolved and washed in ethyl acetate and recrystallised. The final crystals are dissolved in ethylic ether.

The purified product, [1-(2-hydroxy-4,6-diméthoxy-phényl)-méthylidène]-imidazo[1,2-a]pyridine-6-carbohydrazide (compound 1), is a brownish solid (307 mg , yield 91 %).
Melting point = 251-253 °C.
RMN ₁H (DMSO-d₆, 300 MHz) δ : 3.77 (s, 3H), 3.82 (s, 3H), 6.14 (s, 2H), 7.68 (s, 3H), 8.10 (s,1H), 8.81 (s, 1H), 9.21 (s, 1H), 12.10 (s, 1H), 12.34 (s, 1H)
MS (ESI+) *m*/*z*: 341.0 [M+H]+

### Example 1.2 - Preparation of 1-methyl [1-(2-hydroxy-4,6-dimethoxy-phenyl)-methylidene]-1H-indole-5-carbohydrazine (compound 2)

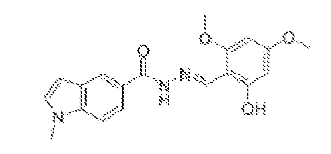

### Preparation of 1-methyl 1H-indole-5-carboxylic acid hydrazide

To 1.0 g (5.3 mmol) of the 1-methyl 1H-benzimidazole 5-carboxylic acid methyl ester, dissolved in 20 ml of ethanol, 20 ml (400mml) of hydrated hydrazine (100%) are added. The solution is heated for 18 hours

After concentration, the concentrate is dissolved in water, alkanized with ammoniac (20%) to basic pH and filtrated the concentrate is then washed with water and vacuum desiccated. 240 mg of a white powder is obtained (R=24%).
RMN 1H (DMSO-d6, 300 MHz) δ : 3.79 (s, 3H), 4.43 (bs,2H), 6.49 (d, J=2.9 Hz, 1H), 7.38 (d, J=3.1 Hz,1H), 7.44 (d, J=8.6 Hz, 1H), 7.66 (dd, J=8.6 Hz, J=1.5 Hz, 1H), 8.08(d, J= 1.5 Hz,1H), 9.60 (bs, 1H).

### Preparation of 1-methyl [1-(2-hydroxy-4, 6-dimethoxy-phenyl)-méthylidene]-1H-indole-5-carbohydrazine

Into a flask equipped with magnetic stirring, 95 mg of 1-methyl 1H-indole-5-carboxylic acid hydrazine (0.5 mmol) are dissolved in 5 ml of anhydrous DMF. 110 µl (1.5 mmol) of diisopropylethylamine (DIEA) are added to this solution and stirred for 10 minutes.
91 mg (0.5 mmol) of 4,6- dimethoxysalicylaldehyde are added to the solution and stirred for 18 hour at room temperature. After evaporation of the solvent the solid residue is crystallized in acetonitrile dissolved and washed in ethyl acetate and washed again in diethyl ether.
120 mg of a brownish solid are obtained (R=68%)
Melting point = 215-217 °C
RMN 1H (DMSO-d6, 300 MHz) δ : 3.77 (s, 3H), 3.82 (s,6H), 6.13 (s,2H), 6.56 (d, J=3 Hz, 2H), 7.44 (d, J=3 Hz,1H), 7.55 (d, J=8.6 Hz, 1H), 7.76 (d, J=8.6 Hz, 1H), 8.22 (s,1H), 8.84 (s, 1H), 11.94 (s,1H), 12.57 (s,1H).
MS (ESI+) m/z : 354.0 [M+H] +

### Example 1.3 - Preparation du [l-(2-hydroxy-4,6-dimethoxy-phenyl)-methylidene]-pyridine 2-carbohydrazide (compound 3)

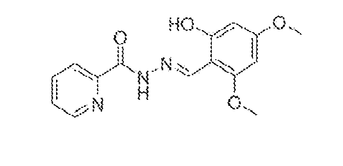

### Preparation of pyridine 2-carboxylic acid hydrazine

To 3.0 g (19.8 mmol) of ethyl picolinate are dissolved in 30 ml of toluene, 4.0 ml (79.8 mmol) of anhydrous hydrazine à 100% are added. This solution is distillated using a Dean Starck distillator for 18 hour.

The solution is concentrated and dissolved in water and brought to basic pH with ammoniac (20%). The precipitate is washed with water and dried. The relating powder (2.3 g) is pinky R=85%).
RMN 1H (DMSO-d6, 300 MHz) δ : 4.50 (bs, 2H), 7.54 (m,1H), 7.96 (m,2H), 8.57 (m, 1H), 9.88 (bs, 1H).
MS (ESI+) m/z : 137.9 [M+H].

### Preparation of [1-(2-hydroxy-4,6-dimethoxy-phenyl)-methylidene]-pyridine 2-carbohydrazide

Into a flask, 137 mg of pyridine 2-carboxylic hydrazine (1.0 mmol) are dissolved in 5 ml of anhydrous DMF. 110 µl (1.5 mmol) of diisopropyléthylamine (DIEA) are added to this solution and stirred for 10 minute.
182 mg (1.0 mmol) of 4,6-dimethoxysalicylaldehyde are added to the solution and maintained at room temperature for 18 hour. The solution is concentrated and crystallized in acetonitrile, washed in ethyl acetate and ethyl ether.
The resulting crystals (180 mg) are brownish R=60%.
Melting point = 185-188 °C
RMN 1H (DMSO-d6, 300 MHz) δ : 3.77 (s, 3H), 3.81 (s,3H), 6.13 (s,2H), 6.12 (s, 2H), 7.64 (m, 1H), 8.03 (m, 2H), 8.70 (d, J=4.6 Hz, 1H), 9.05 (s,1H), 12.51 (s, 1H), 12.57 (s,1H)
MS (ESI+) m/z : 302.0 [M+H] +

### Example 2 : Therapeutical activity

### 2.1 - Cell culture

Several lines of permanent cells may be used in order to demonstrate the effect of the compounds of the invention on the captation and accumulation of lipids in intracellular vesicles. These cells may incorporate lipoproteins, modified lipoproteins, oxidized or acetylated for example, triglycerides or free fatty acids and chylomicrons. These cells have the capacity to transform themselves into foam cells and many, therefore, present an atherogenic phenotype. It is possible to use as an example, THP1, U937, KG1 cells or any other cells capable of being activated and differentiated such as endothelial cells, monocytes/macrophages, smooth muscle cells, or adipocytes in the presence of a medium containing lipoproteins.

Other types of cells having been genetically modified in order to express specific membrane receptors for lipoproteins or fatty acids may be used.
These membrane receptors may form part of the family of scavenger molecules containing proteins such as SRAI, SRAII, SRBI, CD36, LOX1 or members of fatty acid receptors family FABP.

In the present example, cells of the THP1 cell type differentiated under the action of phorbol 12-myristate-13-acetate (PMA) at a concentration à 10⁻⁷ M, were used in order to measure the formation and accumulation of lipid vesicles observed during the formation of foamy macrophages in the presence or absence of compounds 1, 2 and 3.

The cells are cultivated in 96 well plates, at a density of 1,2 àr 5x 10⁵ cells/ml in RPMI-1640 medium or in NEM medium containing 1%, 2%, 5% or 10% of foetal calf serum (FCS), 100 unit / ml of penicillin, 100 µg/ml of streptomycin, 200 mM of L-Glutamine at 37°C in a CO2 incubator. The culture medium is replaced every two days.

The accumulation of lipid vesicles within the cell was measured using THP 1 cells following fixation with paraformaldehyde in PBS medium using a solution containing a fluorescent marker of the Oil Red O type in order to visualize the vesicles. The image of the cells rich in vesicles was analyzed using a microscope equipped with a CCD camera and software necessary for the analysis.

The THP1 cells EACC (5. 10⁵ cells /ml) were maintained and cultivated in RPMI-1640 medium containing 10% foetal calf serum (FCS), 200 mM of L-Glutamine, 100 units/ml of penicillin and 100 µg/ml of streptomycin (Invitrogen-life Technologies at 37°C, in an incubator with 5% CO2. The medium was replaced every 2-3 days.

In order to induce differentiation of the THP1, 1.25 10⁵ cells/well were deposited in the wells of a 96 wells culture plate, in their culture medium containing10⁻⁷ of phorbol-12-myristate-13-acetate (Sigma) for 24 hours at 37°C, 5% CO2.

The differentiated THP1 were subsequently incubated with oxLDL couple with cyanide-3 (1.5 µg/ml) in the presence or absence the compound tested (concentration between 10⁻⁵ M and 10⁻⁹ M) for 24 hours at 37°C, 5% CO2. After fixation of the cells with 4% paraformaldehyde the nuclei were marked with Hoescht 33342 (10 µg/ml) for 20 minutes at ambient temperature. After two washings, 16 image/well of the signal to cyanine-3 and Hoescht 33342 were taken using a fluorescence microscope coupled with a CCD camera. Each image was analyzed and quantified with a MetaMorphsoftware (Universal Imaging).

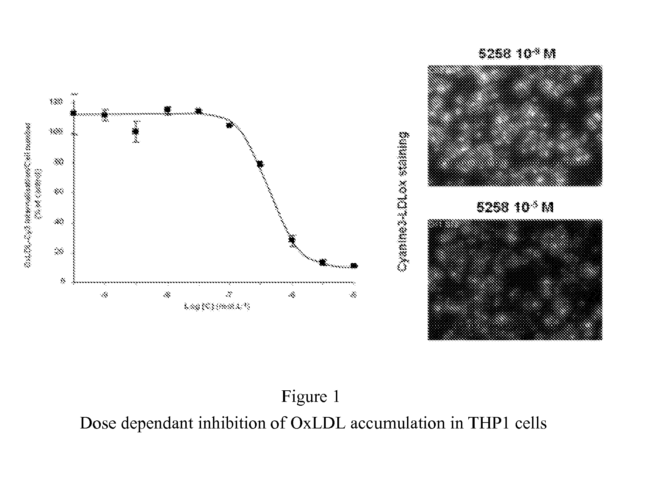

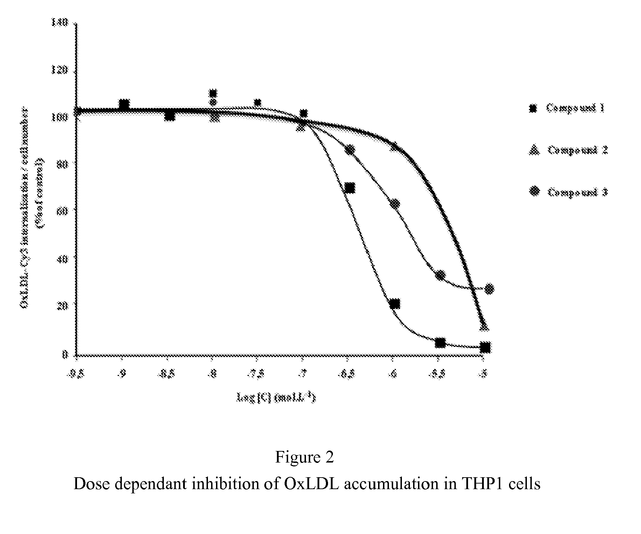

The results obtained with compounds 1, 2 and 3 are reported in Figures 1 and 2.

These results demonstrate that compounds of the present invention are able to reduce the formation of intracellular lipid loaded vesicles in a dose dependent manner.

### 2.2 - Treatment of prediabetic rats

Male obese Zucker diabetic fatty (ZDF *(fa*/*fa)* rats were used. This animal model develops hyperglycemia and insulin resistance.

In this example, six-week-old male prediabetic ZDF rats (Charles River) are provided rat chow and water *ad libidum.* The obese rats are divided into a control and a treated groups (n=6 per subgroup).

The compound 1 is dissolved in PEG 300 and administrated by IP injection at a dose of 10 mg/kg once daily for a period of three weeks.

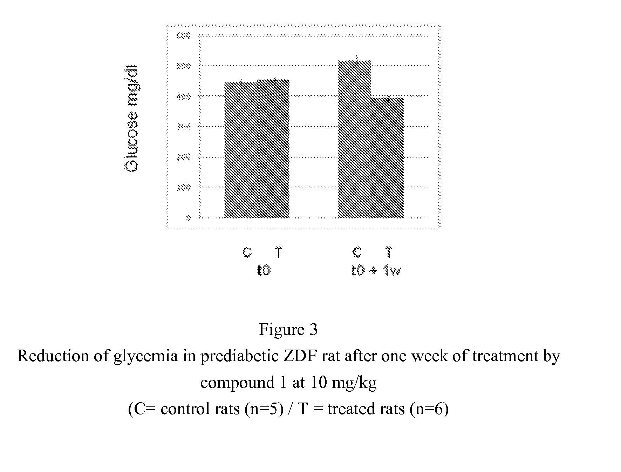

Glucose plasma level can be measured by the commercially available kit using the glucose oxydase method (Sigma Chemical Co.). The results obtained are reported in Figure 3. These results demonstrate that compound 1 significantly reduces the plasma level of glucose in a diabetic animal model.

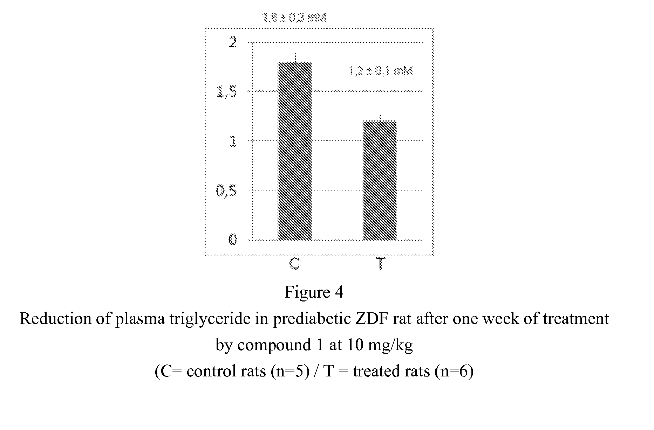

Assay of serum triglycerides may be performed enzymatically using a commercially available kit. In summary, the triglycerides are treated with a lipase in order to generate glycerol and fatty acids. In the presence of ATP, the glycerol is transformed by glucokinase into glycerol-3-phosphate. The glycerol-3-phosphate is subsequently transformed into dihydroxyacetone in generating oxygenated water (H202) which may be detected by formation of quinoneimine in the presence of parachlorophenol, amino-4-antipyrin and peroxydase. The intensity of the quinoneimine coloration is subsequently measured at 505 nm. This coloration is proportional to the quantity of triglycerides present in the sample. The results obtained are reported in Figure 4. These results demonstrate that compound 1 is able to significantly reduce the circulating triglycerides in an obese and diabetic animal model.

### Example 3: In vitro gene mutation test

Gene mutation can be evaluated *in vitro,* using amino-acid requiring strains of *Salmonella typhimuriu.* Different protocols for this test have been published (Ames B.N. et al Mutation Res. 1975, 31, 347-364*, and* Gatehouse D. et al Mutation Res. 1994, 312, 217-233*).* The principle of this bacterial reverse mutation test is that it detects mutations which revert mutations present in the test strains and restore the functional capability of the bacteria to synthesise an essential amino acid. The revertant bacteria are detected by their ability to grow in the absence of the amino acid required by the parent test strain.

In the present example, the guidelines OECD TG 471 (i.e. AMES Test) was used to detect gene mutation at different doses in the TA100, TA98, TA 102, TA 1537 and TA 1535 .

Treatments covering a broad range of concentrations, separated by narrow intervals, were performed both in the absence and presence of metabolic activation (S-9) from Aroclor 1254 induced animals. The compound was formulated in anhydrous analytical grade dimethylsulphoxide (DMSO) and the highest concentration used in was determined following a preliminary cytotoxicity Range-Finder Experiment.

The results obtained are reported in the following Table 1 :

**Table 1 : In vitro gene mutation assay using the AMES test at a non cytotoxic range of concentration between 5 to 1000 µg/plate**

| Ames Assay on bacterial strain | Compound 1 | | Compound CGP 02-01 of WO 2005/082882 | |
|---|---|---|---|---|
| | - S9 | + S9 | - S9 | + S9 |
| TA98 | - | - | - | + |
| TA100 | - | - | - | + |
| TA 102 | - | - | - | - |
| TA1535 | - | - | - | - |
| TA1537 | - | - | - | - |

These results demonstrate that compound 1 does not exhibit mutagenic activity in comparison to compound CGP02-01 disclosed in WO 2005/082882.

## Claims

1. A compound of general formula (I) : 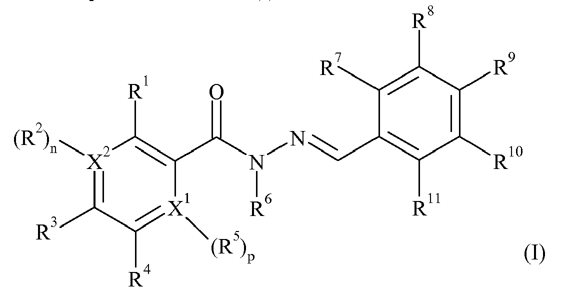 in which :
- X¹ and X² are chosen independently of each other as being a carbon atom or a nitrogen atom;
- n and p are chosen independently of each other as being 0 or 1;
- R¹, R⁴ and R⁵ are chosen independently of each other as being a hydrogen atom, a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
- R² and R³ are chosen independently of each other as being a hydrogen atom, a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
or R² and R³ may together form a 3-, 4-, 5- or 6-membered carbocycle or heterocycle optionally substituted with one or further substituants chosen among a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
- R⁶ is chosen as being a hydrogen atom, a hydroxy group or a C₁-C₈-alkyl;
- R⁷, R⁸, R⁹, R¹⁰ and R¹¹ are chosen independently of each other as being a hydrogen atom, a halogen atom, a hydroxy group, a carboxyl group, -OR¹² or - COR¹², in which R¹² represents an amino group, a C₁-C₈-alkyl or ―NR¹³R¹⁴ in which R¹³ and R¹⁴, are chosen independently of each other as being a halogen atom, a C₁-C₈-alkyl, an aryl radical or R¹³ and R¹⁴ may together form a 3-, 4-, 5- or 6-membered non-fused heterocycle optionally substituted with one or further substituants chosen among a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl;
as well as its pharmaceutically acceptable salts.

2. A compound according to claim 1, **characterised in that** R¹, R⁴, R⁵ and R⁶ are a hydrogen atom.

3. A compound according to claim 1 or 2, **characterised in that** R² is a hydrogen atom.

4. A compound according to any of the claims 1 to 3, **characterised in that** R³ is chosen as being a hydrogen atom or a trifluoromethyl group.

5. A compound according to claim 1 or 2, **characterised in that** R² and R³ form together a phenyl, a pyridine moiety, a pyrimidine moiety, a pyrazine moiety, a pyridazine moiety, a pyrrole moiety, an imidazole moiety or a pyrazole moiety, each of which being optionally substituted with one or further substituants chosen among a halogen atom, a nitro group, an amino group, a cyano group, a hydroxy group, a carboxyl group, a C₁-C₈-alkyl, a C₂-C₈-alkenyl, a C₃-C₈-alkynyl, a C₁-C₆-halogenoalkyl having 1 to 5 halogen atoms, a C₁-C₈-alkylamino, a C₁-C₈-alkoxy, a C₁-C₆-halogenoalkoxy having 1 to 5 halogen atoms, a C₁-C₈-alkylthio, a C₁-C₆-halogenoalkylthio having 1 to 5 halogen atoms, a C₂-C₈-alkenyloxy, a C₂-C₈-halogenoalkenyloxy having 1 to 5 halogen atoms, a C₃-C₈-alkinyloxy, a C₃-C₈-halogenoalkinyloxy having 1 to 5 halogen atoms, or a C₃-C₈-cycloalkyl.

6. A compound according to any of the claims 1 to 5, **characterised in that** R⁷, R⁹ and R¹¹ are chosen independently of each other as being a hydrogen atom, a halogen atom, a hydroxy group or -OR¹² in which R¹² is methyl or ethyl.

7. A compound according to any of the claims 1 to 6, **characterised in that** R⁸ and R¹⁰ are chosen independently of each other as being a hydrogen atom, a halogen atom or a hydroxy group.

8. A compound according to claim 1 having the general formula (I-a) : 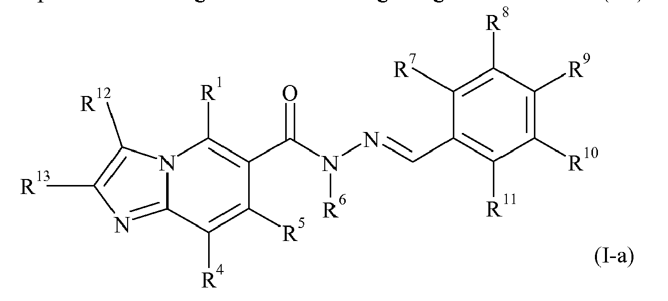 in which :
- R¹, R⁴, R⁵, R⁶, R⁷, R⁸, R⁹, R¹⁰and R¹¹ are as defined in any of the claims 1 to 7 ; and
- R¹² and R¹³ are chosen independently of each other as being a hydrogen atom, an amino group, a hydroxy group, a C₁-C₈-alkoxy, a group of formula (CH₂)ᵣ-(Y)ₛ-(CH₂)t-Z in which r and t are chosen independently of each other as being 0, 1, 2, 3, 4 or 5; s is 0 or 1; Y is chosen as being an oxygen atom, an amino group, a carboxy group and Z is chosen as being a C₁-C₈-alkyl such as methyl or ethyl group, or a C₁-C₆-halogenoalkyl having 1 to 5 halogen atoms such as trifluoromethyl group.

9. A compound according to claim 1 having the general formula (I-b) : 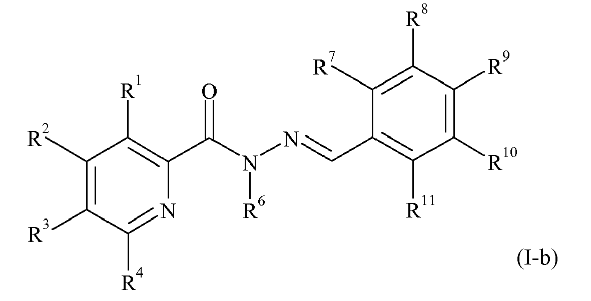 in which R¹, R², R³, R⁴, R⁶, R⁷, R⁸, R⁹, R¹⁰ and R¹¹ are as defined in any of the claims 1 to 7.

10. A compound according to claim 1 having the general formula (I-c) : 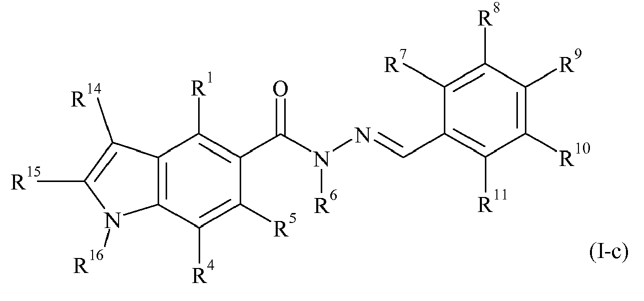 in which :
- R¹, R⁴, R⁵, R⁶, R⁷, R⁸, R⁹, R¹⁰ and R¹¹ are as defined in any of the claims 1 to 7; and
- R¹⁴, R¹⁵ and R¹⁶ are chosen independently of each other as being a hydrogen atom, an amino group, a hydroxy group, a C₁-C₈-alkoxy, a group of formula (CH₂)ᵣ-(Y)ₛ-(CH₂)t-Z in which r and t are chosen independently of each other as being 0, 1, 2, 3, 4 or 5; s is 0 or 1; Y is chosen as being an oxygen atom, an amino group, a carboxy group and Z is chosen as being a C₁-C₈-alkyl such as methyl or ethyl group, or a C₁-C₆-halogenoalkyl having 1 to 5 halogen atoms such as trifluoromethyl group.

11. A process for the preparation of a compound according to any of the claims 1 to 10 according to the following reaction scheme : 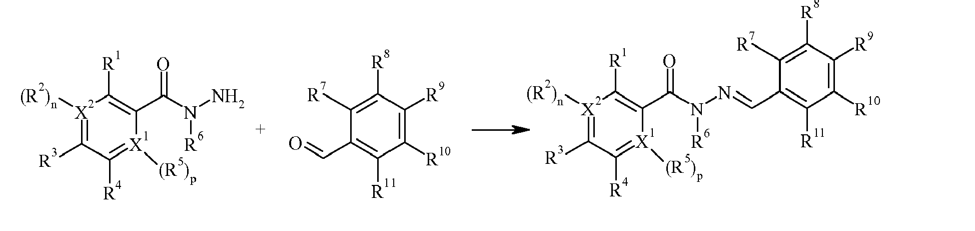 in which R¹ to R¹¹, X¹, X², n and p are as defined above;
wherein said process is conducted in an aprotic solvent, in the presence of a tertiary amine base and at a temperature of from 0°C to 50°C.

12. A pharmaceutical composition comprising, as an active ingredient, a pharmaceutically effective amount of a compound according to any of the claims 1 to 10.

13. A pharmaceutical composition according to claim 12 for the treatment or the prevention of diseases associated with lipid metabolism disorders.

14. A pharmaceutical composition according to claim 12 for the treatment or the prevention of hypercholesterolaemia, hypertriglyceridaemia, dyslipoproteinaemia, chylomicronaemia, lipodystrophy and hyperglycaemia.

15. A pharmaceutical composition according to claim 12 for the treatment or the prevention of obesity, type II diabetes mellitus, insulin resistance, impaired glucose tolerance or associated cardiovascular diseases.

16. A pharmaceutical composition according to claim 12 for the treatment or the prevention of liver steatosis and lipid vesicle accumulation.

17. A pharmaceutical composition according to claim 12 for the treatment or the prevention of restenosis.
